# EUROPEAN PATENT APPLICATION

(11) **EP 1 254 887 A1**
(43) Date of publication of application: **06.11.2002**
(21) Application number: 02253066.1
(22) Date of filing: 30.04.2002
(51) Int. Cl.: C07C 67/39, C07C 69/54, C07C 1/20, C07C 11/09, C07C 29/10, C07C 31/04, B01J 19/00

(54) **Process for producing methyl methacrylate**

(30) Priority: 02.05.2001 JP 2001135628; 21.09.2001 JP 2001288771
(71) Applicant: Sumitomo Chemical Company, Limited, Chuo-ku Osaka 541-8550 (JP)
(72) Inventor: Inoue, Go, Niihama-shi, Ehime (JP); Baba, Katsuo, Singapore 239918 (SG); Moritou, Takayuki, Niihama-shi, Ehime (JP)
(74) Representative: Cresswell, Thomas Anthony

(57) **Abstract**

A process for producing a methyl methacrylate is provided, which comprises the steps of (i) decomposing methyl t-butyl ether to obtain isobutylene and methanol, (ii) oxidizing the isobutylene to obtain at least one compound selected from methacrylic acid and methacrolein and (iii) esterifying at least one compound selected from the methacrylic acid and the methacrolein with the methanol to produce methyl methacrylate. The process of the present invention successfully results in effective use of methanol resources.

## Description

### BACKGROUND OF THE INVENTION

### Field of the Invention

The present invention relates to a process for producing methyl methacrylate. In particular, the present invention relates to aprocess for producing methyl methacrylate comprising a step of esterifying methacrylic acid and/or methacrolein with a methanol which is recovered from a catalytic decomposition of methyl t-butyl ether.

### Description of Related Art

Generally, methyl methacrylate is synthesized by allowing methacrylic acid to react with methanol in the presence of an acid catalyst. Industrial processes of the production thereof are also based on such an esterification reaction. For example, the production process comprises the steps of bringing methacrylic acid and methanol into contact with an esterification catalyst such as a strongly acidic cation exchange resin to react them with each other; and separating the resulting reaction mixture by distillation into a high-boiling component mainly comprising unreacted methacrylic acid and a distillate containing methyl methacrylate and water, which are reaction products, and unreacted methanol. The distillate is cooled and is separated into an oil phase and a water phase. The product, methyl methacrylate, is isolated from the oil phase. The unreacted methanol is recovered from the water phase and is returned to the esterification step, so that methanol is reused. On the other hand, the unreacted methacrylic acid is recovered from the high-boiling component and is also reused in the esterification step. In such a manner, the cost for producing methyl methacrylate may be reduced by recovering the unreacted raw materials, i.e., methanol and methacrylic acid, from the reaction mixture and returning them to the esterification step to reuse them as raw materials therein.

However, in a case that the methanol which is recovered from a production process other than that of methyl methacrylate is reused as it is as a raw material for the esterification to produce methyl methacrylate, the resulting product (methyl methacrylate) may be contaminated with impurities due to the impurities that have been brought together with the reused methanol. Therefore, the obtained methyl methacrylate usually needs to be purified to remove the impurities prior to be reused. This results in the necessity of an additional purification step and the deterioration of a yield of the product, so that the production cost thereof increases. On the other hand, the recovered methanol may be separated from the impurities with purification in advance and then be used as a raw material for the esterification. In that case, however, there are problems such as the purification is complicated and/or costs a lot.

As described above, it has been known that the methanol which is recovered from a production process other than that of methyl methacrylate is difficult to be reused as a raw material for the esterification to produce methyl methacrylate without complicated and/or costly purification. Therefore, in the industrial process, the recovered methanol may or may not be utilized for the production of methyl methacrylate, depending on the complexity and the cost in the purification of the recovered methanol. When there are few advantages in the reuse thereof, there is almost nothing to do except to use the recovered methanol as a fuel for heat recovery, which results in lost of a methanol resource.

### SUMMARY OF THE INVENTION

The inventors of the present invention have made studied with the object of reuse of methanol for the production of methyl methacrylate, even if the methanol is a methanol which has been recovered from a production process other than that of methyl methacrylate. As a result, the inventors have found that a methanol which has been recovered from catalytic decomposition of methyl t-butyl ether can be reused for the production of methyl methacrylate in a simple manner. The present invention has been accomplished on the basis of this finding.

By the catalytic decomposition of methyl t-butyl ether, is provided isobutylene as well as methanol. By catalytic vapor phase oxidation of isobutylene, are provided methacrylic acid and methacrolein, each of which can be used as a raw material for the production of methyl methacrylate together with methanol. In the present invention, the methanol recovered from the catalytic decomposition of methyl t-butyl ether can be utilized in the production of methyl methacrylate, which means that all the products produced by the catalytic decomposition of methyl t-butyl ether can be effectively utilized.

The present invention provides a process for producing a methyl methacrylate, the process comprising the steps of:
(i) decomposing methyl t-butyl ether to obtain an isobutylene and a methanol,
(ii) oxidizing the isobutylene to obtain at least one compound selected from a methacrylic acid and a methacrolein and,
(iii) esterifying at least one compound selected from the methacrylic acid and the methacrolein with the methanol to produce a methyl methacrylate.

The present invention also provides an apparatus for producing a methyl methacrylate, the apparatus comprising:
(a) a reactor for catalytic decomposition of methyl t-butyl ether to obtain a reaction mixture containing an isobutylene and a methanol,
(b) separation means for separating the isobutylene from the reaction mixture,
(c) recovery means for recovering the methanol from the reaction mixture,
(d) a reactor for oxidation of the isobutylene to obtain at least one compound selected from a methacrylic acid and a methacrolein and
(e) a reactor for esterification of at least one compound selected from the methacrylic acid and the methacrolein with the methanol.

### BRIEF DESCRIPTION OF DRAWINGS

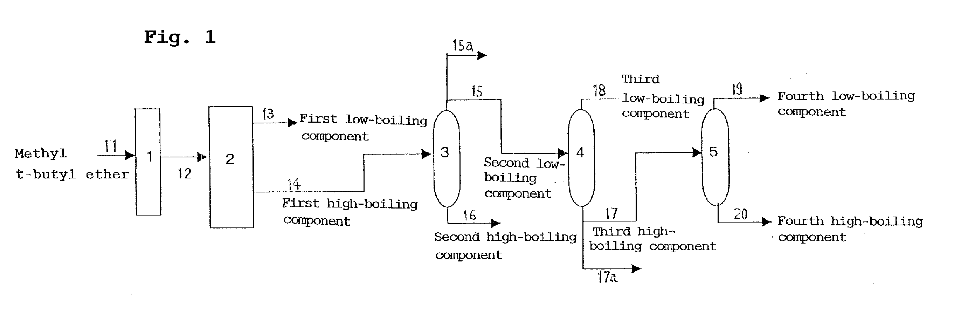

Fig. 1 is a flow diagram illustrating a step of recovering methanol from a reaction mixture obtained by catalytic decomposition of methyl t-butyl ether.

### DETAILED DESCRIPTION OF THE INVENTION

In the present invention, a methyl methacrylate is produced in a process comprising the steps of:
(i) decomposing methyl t-butyl ether to obtain an isobutylene and a methanol,
(ii) oxidizing the isobutylene to obtain at least one compound selected from a methacrylic acid and a methacrolein and,
(iii) esterifying at least one compound selected from the methacrylic acid and the methacrolein obtained in step (ii) with the methanol obtained in step (i) to produce a methyl methacrylate.

The catalytic decomposition of methyl t-butyl ether and the catalytic vapor phase oxidation of the isobutylene are not limited and may be conducted in a known method. Also, the esterification of the methacrylic acid and/or the methacrolein is not limited and may be conducted in a known manner as long as the methanol recovered from the decomposition of methyl t-butyl ether is utilized. The esterification may be conducted in the presence of an esterification catalyst such as an acid or a cation exchange resin so as to provide methyl methacrylate in a high yield.

The process of the present invention may further comprise a step of separating the isobutylene from the decomposition reaction mixture prior to the oxidation step of the isobutylene. Moreover, the process of the present invention may comprise a step of recovering a methanol from the remaining mixture obtained after the separation of the isobutylene, prior to the esterification step.

The recovered methanol may contain at least one compound selected from t-butyl alcohol, water and methyl t-butyl ether. Also, the recovered methanol preferably contains 95 % by weight or more of methanol based on the recovered methanol.

The recovered methanol may be utilized as it is for esterification step, or may be utilized after purified. By conducting the purification step of the recovered methanol, methyl methacrylate with an extremely slight amount of impurities can be produced easily.

The purified methanol may contain about 95 % by weight or more, preferably about 99 % by weight or more, more preferably 99.8 % by weight or more and most preferably 99.9 % by weight or more, of methanol based on the purified methanol.

Specifically, the recovered methanol and/or the purified methanol may be any component selected from the following components (I) to (IV):
(I) a component that contains about 95% by weight or more of methanol based on the component (I) and further contains t-butyl alcohol, water and methyl t-butyl ether;
(II) a component that is obtained by separating methyl t-butyl ether from component (I) and contains about 95% by weight or more, preferably about 98 % by weight or more, of methanol based on the component (II);
(III) a component that is obtained by separating t-butyl alcohol and water from component (I) and contains about 95% by weight or more, preferably about 98 % by weight or more, of methanol based on the component (III);
(IV) a component that is obtained by separating t-butyl alcohol, water and methyl t-butyl ether from component (I) and contains about 99% by weight, preferably 99.8 % by weight or more and more preferably 99.9 % by weight or more of methanol based on the component (IV).

Component (IV) may contain about 10 ppm by weight or less of t-butyl alcohol and/or about 10 ppm by weight or less of methyl t-butyl ether.

The separation of the isobutylene, the recovery of the methanol and the purification of the recovered methanol may be conducted in a known method such as distillation, layer separation (for example, pervaporation) and solvent extraction. Among them, distillation is preferred.

The present invention further provides an apparatus for producing a methyl methacrylate, the apparatus comprising:
(a) a reactor for catalytic decomposition of methyl t-butyl ether to obtain a reaction mixture containing an isobutylene and a methanol,
(b) separation means for separating the isobutylene from the reaction mixture,
(c) recovery means for recovering the methanol from the remaining mixture obtained after the separation of the isobutylene,
(d) a reactor for oxidation of the isobutylene to obtain at least one compound selected from a methacrylic acid and a methacrolein and
(e) a reactor for esterification of at least one compound selected from the methacrylic acid and the methacrolein with the methanol.

By recovery means (c), the methanol having a concentration of 95 % by weight may be obtained. Specifically, the above described the components (I) - (IV) may be obtained by recovery means (c). The components (I) - (IV) may be utilized as the methanol in the reactor (e).

When methyl methacrylate is produced with the apparatus of the present invention, methyl methacrylate with high purity almost free from impurities can be obtained and the cost for the production may be very low.

With reference to Fig. 1, a recovery of methanol in the preset invention may be described as below, which should not be construed as a limitation upon the scope of the present invention. Fig. 1 is a flow diagram illustrating a step in which methanol is recovered from a reaction mixture obtained by the catalytic decomposition of methyl t-butyl ether.

The methanol to be recovered in Fig. 1 is produced in the catalytic decomposition of methyl t-butyl ether. The catalytic decomposition of methyl t-butyl ether can provide an isobutylene without any problem, while the decomposition reaction mixture may contain methanol, isobutylene, t-butyl alcohol, water and unreacted methyl t-butyl ether as well as isobutylene. It is preferred that, from the reaction mixture, the isobutylene is separated first and then the methanol is separated and recovered. The recovered methanol is reused as a raw material for esterification.

In Fig. 1, methyl t-butyl ether is supplied through methyl t-butyl-ether supply line 11. The methyl t-butyl ether is catalytically decomposed in reactor 1 (reactor for catalytic decomposition) to obtain a reaction mixture (catalytic decomposition step). The reaction mixture is then supplied to isobutylene separation column 2 (isobutylene separation means) through catalytic decomposition reaction product supply line 12.

In isobutylene separation column 2, the reaction mixture is separated into a first low-boiling (light) component comprising isobutylene and a first high-boiling (heavy) component comprising methanol, t-butylalcohol, water and methyl t-butyl ether (isobutylene separation step). The component comprising isobutylene may be discharged from isobutylene separation column 2 through isobutylene drain line 13, followed by being oxidized to produce a methacrylic acid and/or a methacrolein.

The first high-boiling (heavy) component is supplied to methanol recovery column 3 (methanol recovery means) through first high-boiling component supply line 14 (methanol recovery step). In methanol recovery column 3, the first high-boiling (heavy) component is separated into a second low-boiling (light) component containing methanol and methyl t-butyl ether and a second high-boiling (heavy) component containing methanol, t-butyl alcohol and water.

The second low-boiling component is contaminated with methyl t-butyl ether, and may contain 95% by weight or more of methanol based on the second low-boiling component. The methyl t-butyl ether contained in the second low-boiling component may be easily separated by distillation from methyl methacrylate, which is generated after the esterification of methacrylic acid and/or methacrolein with methanol. Therefore, the second low-boiling (light) component may be used as a methanol for the esterification as it is. For example, the second low-boiling component is discharged from second low-boiling component drain line 15a and is used for the esterification with the methacrylic acid and/or the methacrolein. On the other hand, the second high-boiling component may be discharged through second high-boiling component drain line 16.

For producing methyl methacrylate containing a smaller amount of impurities, it is preferred to purify the second low-boiling (light) component before the esterification step so as to remove the methyl t-butyl ether contained therein. In such a preferable case , the second low-boiling (light) component is supplied to methanol-purifying distillation column 4 (methanol purification means) through second low-boiling component supply line 15. In methanol-purifying distillation column 4, the second low-boiling component may be separated by distillation into a third low-boiling (light) component comprising methanol and methyl t-butyl ether and a third high-boiling (heavy) component comprising 99.8% by weight of methanol based on the third high-boiling (heavy) component. The third high-boiling (heavy) component may be used for the esterification as it is. For example, the third high-boiling component is discharged from third high-boiling component drain line 17a and is used for the esterification with the methacrylic acid and/or the methacrolein. The third low-boiling component may be discharged from third low-boiling component drain line 18.

The third high-boiling (heavy) component may be contaminated with traces of t-butyl alcohol and water. It is possible to remove these contaminants by purification to a methanol with a very high purity before utilized as a raw material for the esterification. In such a preferable case, the third high-boiling (heavy) component is supplied to methanol-purifying distillation column 5 through third high-boiling component supply line 17. In methanol distillation column 5 (methanol purification means), the third high-boiling (heavy) component is separated by distillation into a fourth low-boiling (light) component comprising 99.9% by weight or more of methanol and a fourth high-boiling (heavy) component comprising t-butyl alcohol and water. When the fourth low-boiling (light) component is used for the esterification, a methyl methacrylate containing an extremely slight amount of impurities can be obtained. In such a preferable case, the fourth low-boiling component is discharged through fourth low-boiling component drain line 19 and is used for esterification. On the other hand, the fourth high-boiling component is discharged through fourth high-boiling component drain line 20.

In the method of recovering methanol which is described above with reference to Fig. 1, t-butyl alcohol and water are removed; unreacted methyl t-butyl ether is then removed; and then again t-butyl alcohol and water are removed, so as to recover and purify the methanol for the esterification. The order of the removal of these impurities in the methanol recovery/ purification method is not restricted to the above-described order. The order may be changed and any removal step may be omitted.

The components (I)' - (IV)' presented below may be used as a methanol for the esterification.
(I)' A component that contains about 95% by weight or more of methanol based on the component (I)' and further contains t-butyl alcohol, water and methyl t-butyl ether.
(II)' A component that is obtained by separating methyl t-butyl ether from component (I)' and contains about 95% by weight or more, preferably about 98 % by weight or more, of methanol based on the component (II)'.
(III)'A component that is obtained by separating t-butyl alcohol and water from component (I)' and contains about 95% by weight or more, preferably about 98 % by weight or more, of methanol based on the component (III)'.
(IV)' A component that is obtained by separating t-butyl alcohol, water and methyl t-butyl ether from component (I)' and contains about 99% by weight, preferably 99.8 % by weight or more and more preferably 99.9 % by weight or more of methanol based on the component (IV)'.
   A methacrylic acid and a methacrolein, each of which is another raw material for the production of methyl methacrylate, may be obtained in a known method by catalytic vapor phase oxidation of the isobutylene and/or t-butyl alcohol. Isobutylene can be obtained by decomposition of methyl t-butyl ether. The methacrylic acid and a methacrolein may be obtained in a known method by catalytic vapor phase oxidation of t-butyl alcohol and/or isobutene. Also, a methacrylic acid may be obtained in a known method by catalytic vapor phase oxidation of methacrolein or isobutyl aldehyde.
   For the esterification of a methacrylic acid and/or a methacrolein with a methanol, an esterification catalyst such as an acid and a cation exchange resin may be used. Preferably, a strongly acidic cation exchange resin is used. Examples of the strongly acidic cation exchange resin include a porous strongly acidic cation exchange resin, for example, DUOLITE C-26CH (manufactured by Sumitomo Chemical Co., Ltd.).
   The esterification may be carried out, using a suspended bed or fixed bed, in a liquid phase at a temperature of from about 70 °C to about 120 °C. A polymerization inhibitor may be added to the esterification mixture. The polymerization inhibitor to be added may be conventionally known polymerization inhibitor. Examples of the polymerization inhibitor include hydroquinone, phenothiazine and methoquinone.
   The resulting product obtained in the esterification may be purified by distillation in a conventional manner. That is, the esterification mixture may be separated by distillation into a distillate comprising methyl methacrylate and water, both of which are generated by the esterification reaction, and unreacted methanol; and into a high-boiling component mainly comprising unreacted methacrylic acid. The distillate may be separated into an oil phase and a water phase after cooling. From the oil phase, the methyl methacrylate is obtained by distillation. From the water phase, the methanol is recovered by distillation. The recovered methanol may be fed back to the esterification step and be reused. On the other hand, the high-boiling component may be fed back to the esterification step as it is, or is preferably fed back after recovering the methacrylic acid contained therein by distillation.
   As described above, the present invention provide a process for producing methyl methacrylate in which a methanol obtained by catalytically decomposing methyl t-butyl ether is used together with a methacrylic acid and/or a methacrolein. The methacrylic acid and/or the methacrolein may be obtained by catalytically decomposing methyl t-butyl ether, followed by the catalytic vapor phase oxidation of the resulting decomposition product. The process in the present invention successfully results in effective use of methanol resources.
   The invention being thus described, it will be apparent that the same may be varied in many ways. Such variations are to be regarded as within the spirit and scope of the invention, and all such modifications as would be apparent to one skilled in the art are intended to be within the scope of the following claims.
   The entire disclosure of the Japanese Patent Application No. 2001-135628 filed on May 2, 2001 and the Japanese Patent Application No. 2001-288771 filed on September 21, 2001, both indicating specification, claims, drawings and summary, are incorporated herein by reference in their entirety.
   The present invention is described in more detail by following Examples , which should not be construed as a limitation upon the scope of the present invention.

### Example 1

The catalytically decomposition of methyl t-butyl ether was carried out to obtain a reaction mixture containing isobutylene and methanol. The reaction mixture was separated by distillation into a first high-boiling component and a first low-boiling component. The first high-boiling component was then separated by distillation into a second high-boiling component and a second low-boiling component. The second low-boiling component contained a 98% by weight of methanol, 0.9% by weight of methyl t-butyl ether, 0.09% by weight of t-butyl alcohol, several % by weight of inert components and a small amount of water.

A distillation device mainly made of glass was prepared. The distillation device had a distillation column, a 2-L glass flask (connected to the column at its bottom) and a condenser (connected to the column at its top) cooling with water. The distillation column was a cylindrical glass column (inner diameter: 30 mm) which was packed with a Dickson packing with a diameter of 3 mm to a height of 120 cm (so that the theoretical plate number is 23). Using the distillation device, methyl t-butyl ether was removed by distillation from the second low-boiling component as follows.

The operating pressure was set to atmospheric pressure during the distillation. Heating of the distillation column was carried out by heating the bottom of the flask with an oil bath. The second low-boiling component was supplied at a rate of 1200 g/h to the distillation column. A part of the distillate condensed in the top condenser was drained at a rate of 6 g/h and the whole amount of the remainder was fed back to the distillation column. The inert components, which were not condensed in the condenser, were discharged from the device at a rate of 29 g/h in the form of vent gas. The bottom liquid was drained from the flask at a rate of 1165 g/h and the air was supplied to the bottom of the column at a rate of 300 ml/h. When the concentrations of methyl t-butyl ether and t-butyl alcohol in a drain obtained from the flask became 2 ppm or less and 0.09% by weight, respectively, the temperature at the top of the column was 62.5 °C, the temperature of the bottom liquid in the flask was 66.5 °C, and the methanol concentration in the drain obtained from the flask was 99.8% by weight.

### Example 2

The same distillation device as used in Example 1 was prepared except that a Dickson packing with a diameter of 3 mm was packed in the distillation column to a height of 150 cm (so that the theoretical plate number is 27). Using the distillation device , the methanol in the drain (third high-boiling component) which had been obtained in Example 1 was purified by as follows.

The third high-boiling component was supplied at a rate of 325 g/h to the distillation column. A distillate condensed in the top condenser was drained at a rate of 318.5 g/h and the remainder of the distillate was fed back to the distillation column. The rate of discharging the bottom liquid from the flask was set to 6.5 g/h and a rate of supplying the air from the bottom of the column was set to the same as in Example 1. When the concentrations of methyl t-butyl ether and t-butyl alcohol in a distillate became 2 ppm or less and 3 ppm, respectively, the temperature at the top of the column was 64.5°C, the temperature of the bottom liquid in the flask was 68.5°C, and the methanol concentration in the distillate was 99.9% by weight.

Production of methyl methacrylate was carried out by performing an esterification of the methanol obtained above as the distillate with a methacrylic acid reagent (special grade, manufactured by Woko Pure Chemical Industries, Ltd.). Specifically, the esterification was carried out as follows.

A solution comprising 20% by weight of the methanol obtained above as the distillate and 80% by weight of the methacrylic acid reagent was prepared. Also, a fixed bed reactor made of 304SS and having 24 mm in inner diameter and 500 mm in height was prepared. The fixed bed was packed with an ion exchange resin DUOLITE (manufactured by Sumitomo Chemical Co., Ltd.) as an esterification catalyst. The solution of the methanol and the methacrylic acid reagent was supplied under the pressure of 245 KPa into the bottom of the fixed bed reactor to conduct the esterification, while maintaining the reactor at a temperature of 80°C. The resulting reaction product was purified by distillation to obtain a methyl methacrylate. The conversion of methacrylic acid to methyl methacrylate was from 47% to 48%. The concentrations of impurities contained in the methyl methacrylate were obtained by gas chromatographic analysis, which are shown in Table 1.

### Comparative Example 1

The same process as in Example 2 was carried out except that, instead of the methanol obtained in Example 2 as the distillate, a special-grade methanol (manufactured by Woko Pure Chemical Industries, Ltd.) was used to obtain a methyl methacrylate. The conversion of methacrylic acid to methyl methacrylate was from 47% to 48%. The concentrations of impurities contained in the methyl methacrylate were obtained by gas chromatographic analysis, which are shown in Table 1.

The results in Table 1 show that, by using a methanol recovered and purified from the catalytic decomposition of methyl t-butyl ether, the same grade of methyl methacrylate which contains a slight amount of impurities was obtained as that obtained using the special-grade methanol reagent.

**Table 1**

| Impurities | Isobutylene (%) | t-Butyl alcohol (%) | Methyl t-butyl ether (%) | Methyl t-butyl ether (%) |
|---|---|---|---|---|
| Example 1 | <0.0001 | <0.0002 | <0.0002 | <0.0002 |
| Comparative Example 1 | <0.0001 | <0.0002 | <0.0002 | <0.0002 |

## Claims

1. A process for producing methyl methacrylate, the process comprising the steps of:
(i) decomposing methyl t-butyl ether to obtain isobutylene and methanol,
(ii) oxidizing the isobutylene to obtain at least one compound selected from methacrylic acid and methacrolein and
(iii) esterifying at least one compound selected from the methacrylic acid and the methacrolein with the methanol to produce methyl methacrylate.

2. A process according to claim 1, further comprising a step of separating the isobutylene from the decomposition reaction mixture prior to the oxidation step of the isobutylene.

3. A process according to claim 2, further comprising a step of recovering methanol from the remaining mixture obtained after the separation of the isobutylene, prior to the esterification step.

4. A process according to claim 3, wherein the recovered methanol comprises 95% by weight or more of methanol based on the recovered methanol and comprises at least one compound selected from t-butyl alcohol, water and methyl t-butyl ether.

5. A process according to claim 3, further comprising a step of purifying the recovered methanol.

6. A process according to claim 5, wherein the purified methanol comprises about 95% by weight or more of methanol based on the purified methanol.

7. A process according to claim 5, wherein the purified methanol comprises about 99% by weight or more of methanol based on the purified methanol.

8. A process according to claim 5, wherein the purified methanol comprises about 99.9% by weight or more of methanol based on the purified methanol.

9. An apparatus for producing methyl methacrylate, the apparatus comprising:
(a) a reactor for catalytic decomposition of methyl t-butyl ether to obtain a reaction mixture comprising isobutylene and methanol,
(b) separation means for separating the isobutylene from the reaction mixture,
(c) recovery means for recovering the methanol from the remaining mixture obtained after the separation of the isobutylene,
(d) a reactor for oxidation of the isobutylene to obtain at least one compound selected from methacrylic acid and methacrolein and
(e) a reactor for esterification of at least one compound selected from the methacrylic acid and the methacrolein with the methanol.

10. An apparatus according to claim 9, wherein the methanol obtained by recovery means (c) has a concentration of about 95% by weight.
